# EUROPEAN PATENT SPECIFICATION

(11) **EP 4 105 663 B1**
(45) Date of publication and mention of the grant of the patent: **02.12.2026**
(21) Application number: 21752929.6
(22) Date of filing: 15.01.2021
(51) Int. Cl.: G01N 35/10, G01N 35/00, G01N 33/53, G01D 5/34, G01N 21/59, G01N 33/543, G01N 21/07

(54) **METHOD FOR AUTOMATICALLY DISTINGUISHING WHOLE BLOOD/PLASMA/NON-SUCTION BY MEANS OF REFLECTIVE PHOTOSENSOR**
VERFAHREN ZUM AUTOMATISCHEN UNTERSCHEIDEN VON VOLLBLUT/PLASMA/NICHTANSAUGEN MIT HILFE EINES REFLEKTIERENDEN PHOTOSENSORS
PROCÉDÉ DE DISTINCTION AUTOMATIQUE DE SANG TOTAL/PLASMA/NON-ASPIRATION AU MOYEN D'UN PHOTOCAPTEUR RÉFLÉCHISSANT

(30) Priority: 10.02.2020 KR 20200015762
(43) Date of publication of application: 21.12.2022
(73) Proprietor: I-SENS, INC., Seoul 06646 (KR)
(72) Inventor: YOU, Jungmin, Seoul 06646 (KR); JUNG, Jae Won, Seoul 06646 (KR); JIN, Yungjoon, Seoul 06646 (KR); KIM, Yu Sung, Seoul 06646 (KR); KIM, Ui Sik, Seoul 06646 (KR); JIN, Woo Seob, Seoul 06646 (KR); HONG, Miin, Seoul 06646 (KR)
(74) Representative: Gevers Patents
(86) International application number: PCT/KR2021/000611
(87) International publication number: WO 2021/162257

(56) References cited:
- JP-A- 2013 501 937
- JP-B2- 4 452 277
- KR-A- 20140 141 879
- KR-A- 20150 104 569
- KR-B1- 101 970 790
- US-A1- 2008 096 285
- US-A1- 2013 065 256
- US-A1- 2017 292 967

## Description

### [Technical Field]

The present invention relates to a method of automatically distinguishing suction or non-suction of whole blood or blood plasma by using a reflective photosensor, and more particualriy, to a method of automatically distinguishing whether whole blood or blood plasma is sucked or whether the whole blood or the blood plasma is not sucked by using a reflective photosensor, the method being capable of accurately checking whether the whole blood or the blood plasma is sucked or whether the whole blood or the blood plasma is not sucked from a round cartridge into a pipette mounted on a tip of an automatic immunoassay device including the round cartridge which may simultaneously perform the centrifugation and automatic analysis of a blood sample and the tip which may be moved up, down, left and right based on the round cartridge.

### [Background Art]

In general, an immunoassay device may measure concentration of a specific biomarker protein in the blood plasma or serum of a human body.

The immunoassay device is not necessary to calibrate the measured concentration before outputting a result when directly using the blood plasma or serum as a measurement sample. However, when using whole blood as the measurement sample and analyzing the blood plasma or serum in the whole blood, the immunoassay device is necessary to correct the measured concentration by using a hematocrit of the measurement sample before outputting the result.

In general, a conventional immunoassay device may require centrifugation of the measurement sample separately performed in an external laboratory, or the manually input measured hematocrit and type (whole blood/blood plasma/serum) of the sample.

In this case, it may be necessary to separately input information on the measurement sample.

In addition, in order to solve this problem, a method has been proposed in which the hematocrit is optically measured to correct the result. However, it is impossible to generally use this method for all the immunoassay devices.

In addition, it may be inconvenient to separately input the information on the measurement sample.

That is, when using the sample of the whole blood, it is necessary to calculate and correct a ratio of a volume occupied by red blood cells to a total volume of the whole blood, i.e. the hematocrit, as a parameter to be considered when analyzing a substance included in a blood plasma component, and this parameter may significantly depend on age, climate, nutrition, illness and another factor. For example, a hematocrit of 40% may indicate that the red blood cells occupy 40% of the volume of the whole blood and the blood plasmas occupy 60% thereof. Therefore, when a volume of the hematocrit is increased in a patient's blood, a volume of the blood plasma may be descreased in the blood sample having a predetermined constant volumem which is injected into the test device, and vice versa.

Only the blood plasma component may contain an analyte to be measured. Therefore, when a reaction mixture gets a smaller volume of the blood plasma component added thereto, the reaction mixture may have a reduced concentration of the substance to be measured therein, thus obtaining a smaller analytical value, and vice versa.

In any and every analysis of the concentration of the blood plasma substance in whole blood, it is necessary to perform a correction for a change in the hematocrit to obtain a true blood plasma concentration.

In order to omit this correction, it is possible to perform an analysis on serum or blood plasma in which the red blood cells are filtered or centrifuged in advance, which may increase the cost due to a complex design. In addition, most of the immunoassay devices may require the manual input of the whole blood, blood plasma or serum, which may not only be very cumbersome for an analyst but also may significantly reduce accuracy of the immunoassay if the analyst makes an error.

US20080096285A1 **discloses a method of determining a kind of a sample, in a method for analyzing a substance by the steps of supplying the sample to be analyzed to a reaction system by a supplying means comprising a transparent region composed of a transparent material, reacting a** reagent **for detecting the substance with the sample in the reaction system, and analyzing a signal derived from a product obtained by the reaction, characterized by irradiating the transparent region with light in the supplying step, and analyzing an optical intensity of the light.**

US 2017/292967 A1 **discloses a modular automatic analyzer using a circular type cartridge enabling centrifugation according to an exemplary embodiment of the present invention includes: a main body frame; an X-axis conveying unit which reciprocally moves in an X-axis direction with respect to the main body frame; a circular type cartridge accommodating housing which is installed with respect to the X-axis conveying unit so as to be reciprocally movable in the X-axis direction by the X-axis conveying unit, a single rotation drive unit which is installed in the circular type cartridge accommodating housing and is rotated step by step at a predetermined interval or rotated at a high speed for centrifugation, a circular type cartridge which is installed at an upper surface of the circular type cartridge accommodating housing so as to be rotatably connected to the rotation drive unit and has wells disposed to be spaced apart from each other at predetermined intervals in a circumferential direction of a disc-shaped main body, a tip lifting unit which picks up and moves a tip upward and downward, a measurement unit which measures a reaction in the well, a Z-axis drive unit which is provided with the tip lifting unit and the measurement unit and reciprocally operates in a Z-axis direction with respect to the main body frame, and a control unit C which controls an automatic immunity analyzer.**

### [Disclosure]

### [Technical Problem]

The present invention relates to a method of automatically distinguishing suction or non-suction of whole blood or blood plasma by using a reflective photosensor, the method being capable of accurately checking whether the whole blood or the blood plasma is sucked or whether the whole blood or the blood plasma is not sucked into a pipette mounted on a tip of an automatic immunoassay device including a round cartridge for simultaneously performing the centrifugation and automatic analysis of a blood sample and the tip which is movable up, down, left and right based on the round cartridge.

### [Technical Solution]

The invention is defined in the appended set of claims.

### [Advantageous Effects]

As set forth above, the method of automatically distinguishing the suction or non-suction of the whole blood or the blood plasma by using the reflective photosensor according to an exemplary embodiment of the present invention may accurately check whether the whole blood or the blood plasma is sucked or whether the whole blood or the blood plasma is not sucked from the round cartridge into the pipette mounted on the tip of the automatic immunoassay device including the round cartridge which may simultaneously perform the centrifugation and automatic analysis of the blood sample and the tip which is movable up, down, left and right based on the round cartridge.

### [Description of the Drawings]

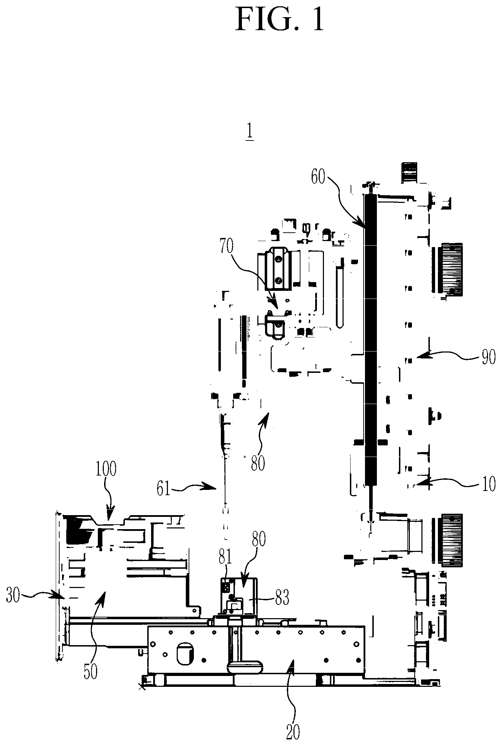

FIG. 1 is a side view of an automatic immunoassay device including a tip which may be moved up, down, left and right based on a conventional round cartridge.

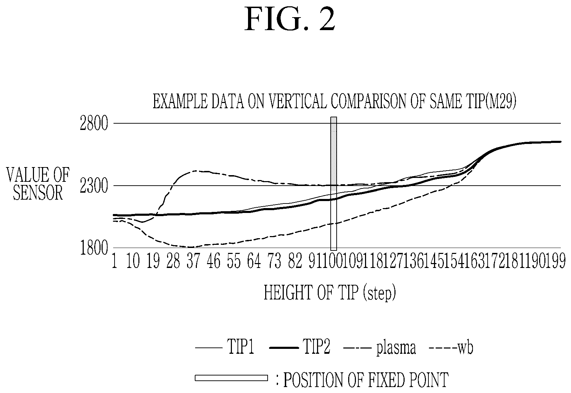

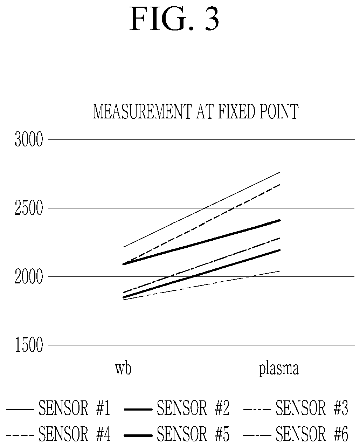

FIGS. 2 and 3 are a graph showing a method of determining whether a type of the blood is whole blood or blood plasma or whether the whole blood or the blood plasma is not sucked, by comparing blood non-suction data with blood suction data in the automatic immunoassay device including the tip which may be moved up, down, left and right based on the conventional round cartridge, and a graph showing a determination result value obtained from the same tip used for one measurement.

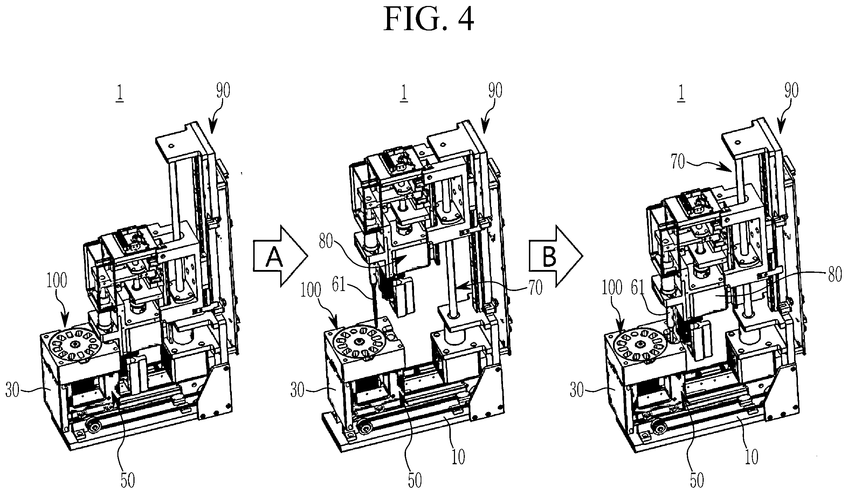

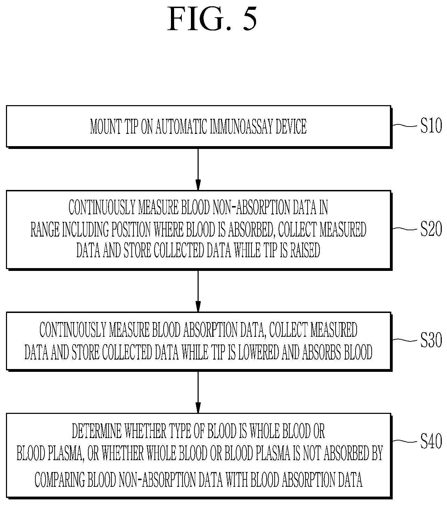

FIG. 4 is a side view showing an operation of the automatic immunoassay device including the tip which may be moved up, down, left and right based on the round cartridge according to an exemplary embodiment of the present invention. FIG. 5 is a flowchart showing a method of comparing the blood non-suction data with the blood suction data in the automatic immunoassay device including the tip which may be moved up, down, left and right based on a round cartridge according to an exemplary embodiment of the present invention to determine whether the type of the blood is the whole blood or the blood plasma, or whether the whole blood or the blood plasma is not sucked.

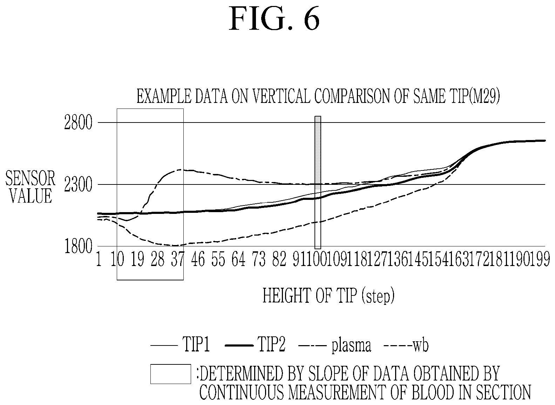

FIG. 6 is a graph showing a method of determining whether the type of the blood is the whole blood or the blood plasma, or whether the whole blood or the blood plasma is not sucked, by comparing the blood non-suction data with the blood suction data in the automatic immunoassay device including the tip which may be moved up, down, left and right based on the round cartridge according to an exemplary embodiment of the present invention.

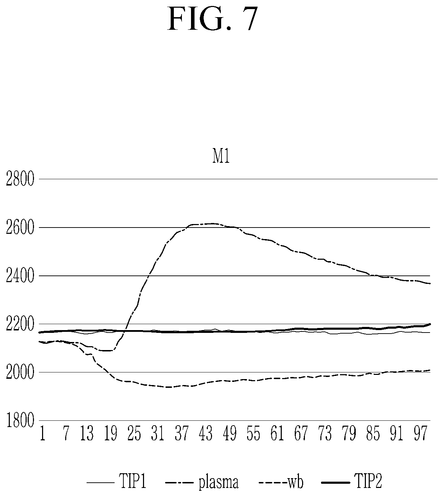

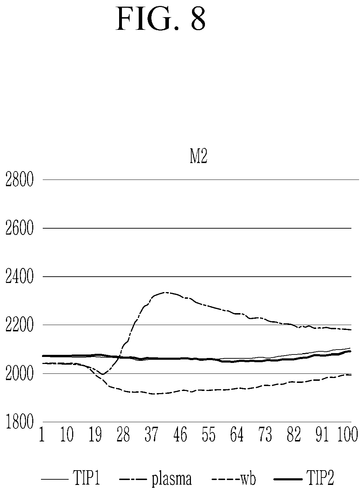

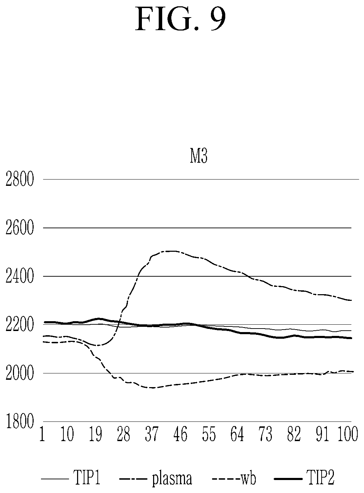

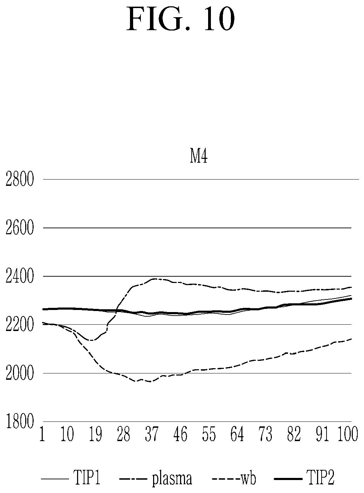

FIGS. 7 to 10 are graphs showing whether the type of the blood is the whole blood or the blood plasma and a result value of the whole blood or blood plasma when the whole blood or the blood plasma is not sucked by comparing the blood non-suction data with the blood suction data, obtained from the same tip used for one measurement, in the automatic immunoassay device including the tip which may be moved up, down, left and right based on the round cartridge according to an exemplary embodiment of the present invention.

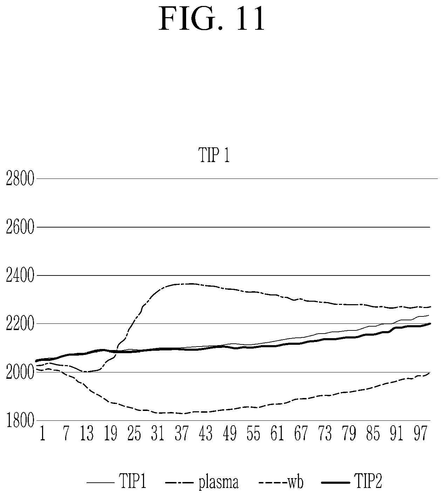

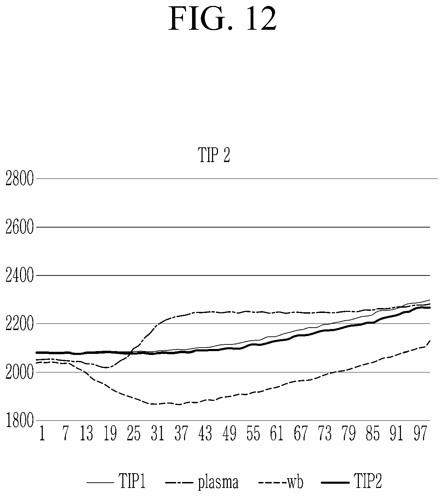

FIGS. 11 and 12 are graphs showing whether the type of the blood is the whole blood or the blood plasma and the result value of the whole blood or blood plasma when the whole blood or the blood plasma is not sucked by comparing the blood non-suction data with the blood suction data, obtained from two different tips used for one measurement, in the automatic immunoassay device including the tip which may be moved up, down, left and right based on the round cartridge according to an exemplary embodiment of the present invention.

### [Mode for Invention]

Hereinafter, exemplary embodiments of the present invention will be described in detail with reference to the accompanying drawings so that those skilled in the art to which the present invention pertains may easily practice the present invention. The present invention may be modified in various different forms, and is not limited to embodiments provided in the present specification. In addition, in the drawings, portions unrelated to the description are omitted to clearly describe the present invention, and similar portions are denoted by similar reference numerals throughout the specification.

In the specification, unless explicitly described to the contrary,"including" any component will be understood to imply the inclusion of other components rather than the exclusion of any other components. In addition, a term "part," "module," "device" or the like, described in the specification may indicate a unit of processing at least one function or operation, which may be implemented by hardware or software or a combination of hardware and software.

Hereinafter, the description describes an automatic analysis device including a cartridge capable of performing the centrifugation and automatic analysis of a blood sample, according to an exemplary embodiment of the present invention, in detail with reference to the attached drawing.

In order to solve problems of the prior art, as shown in FIG. 1, Korean Patent Publication No. 10-1970790, entitled "Method and apparatus with improved accuracy", which is filed and registered by the same applicant, shows an automatic analysis device 1 including a cartridge 100 capable of performing the centrifugation and automatic analysis of the blood sample, in which the cartridge 100 capable of performing the centrifugation and automatic analysis of the blood is installed in a cartridge accommodation housing 30 by reciprocating a main frame 10 disposed at a bottom of a cartridge accommodation housing 30 to an X-axis transfer unit 20 in an X-axis direction, a sample is injected into the cartridge 100 by moving a tip 61 by a tip elevation unit 60 disposed at an upper portion of the device 1, the cartridge is rotated by a rotary drive unit 50 disposed at a lower portion of the device 1 to centrifuge whole blood or rotated to repeatedly suck/discharge a reagent, and an analysis process is performed by using a measurement unit 70.

Here, the main frame 10 may further include a reflective photosensor 80 accommodated in the cartridge accommodation housing 30 and automatically measuring, from the tip 61, whether a type of the blood is the whole blood or the blood plasma, or whether the whole blood or the blood plasma is not sucked.

The reflective photosensor 80 includes a light source 81 and a light receiving element 83. The light source 81 is a long and thin light emitting diode (LED) disposed in an axial direction of the cartridge 100, and the light receiving element 83 is a charge coupled device (CCD) linear image sensor in which the plurality of light receiving elements are arranged in one row in the axial direction. The light receiving element 83 receives light reflected from the light source 81 on a well surface of the cartridge 100.

Here, as shown in FIGS. 2 to 4, when the tip 61 reaches a height of 100 steps, which is half of a total height of 200 steps, i.e., when the tip 61 reaches its fixed point, it is possible to irradiate light from the light source 81 of the reflective photosensor 80, and read an output value of the light receiving sensor 83 reflected from a detected substance. It is then possible to compare a result value obtained from the same tip 61 used for one measurement or different tips 61, with a correction value of whole blood (wb) or the blood plasma to determine whether the type of the blood is the whole blood or the blood plasma, or whether the whole blood or the blood plasma is not sucked.

However, the above-described method of reading the output value of the reflective photo sensor 80 at the fixed point of the tip 61 by using the sensor and comparing the output values of the whole blood and blood plasma with their correction values to make the determination may be difficult to accurately distinguish the values from each other due to deviation in device performance of the light receiving sensor 83 when using the different light receiving sensor 83, as shown in FIG. 3 and Table 1, and may be unable to accurately distinguish the two values from each other because the obtained output values may have an insufficient margin therebetween.

**[Table 1]**

| Sensor no. | Wb sucked | Plasma sucked |
|---|---|---|
| 1 | 2221 | 2274 |
| 2 | 1862 | 2202 |
| 3 | 1840 | 2044 |
| 4 | 2092 | 2679 |
| 5 | 2092 | 2422 |
| 6 | 1906 | 2291 |

As shown in Table 1 and FIG. 3, the light receiving sensors 83, i.e. sensor nos. 1, 2, 3, 4, 5 and 6, may each have a higher measurement value obtained when the whole blood (wb) is sucked than a measurement value obtained when the blood plasma is sucked. However, some sensors may show the measurement value of the whole blood (wb) is the same as the measurement value of the blood plasma (Blood plasma). In this case, the two values may not be clearly distinguished from each other.

In addition, each correction value of the two measurement values is necessary to be pre-determined for performing one measurement, depending on whether the type of the blood is the whole blood or the blood plasma whenever measuring the whole blood or the blood plasma is not sucked into the tip 61.

In addition, the tip is necessary to be replaced for every measurement, and the measurement value may thus also be changed.

Hereinafter, the description describes a method of automatically distinguishing suction or non-suction of whole blood or blood plasma by using a reflective photosensor according to an exemplary embodiment of the present invention in detail with reference to FIGS. 4 and 5.

FIG. 5 is a flowchart showing a method of comparing the blood non-suction data with blood suction data in the automatic immunoassay device including the tip which may be moved up, down, left and right based on a round cartridge according to an exemplary embodiment of the present invention to determine whether the type of the blood is the whole blood or the blood plasma, or whether the whole blood or the blood plasma is not sucked; and FIG. 4 is a side view showing an operation of the automatic immunoassay device including the tip which may be moved up, down, left and right based on the round cartridge according to an exemplary embodiment of the present invention.

As shown in FIGS. 4 and 5, the method of automatically distinguishing suction or non-suction of whole blood or blood plasma by using a reflective photosensor according to an exemplary embodiment of the present invention may provide a constant measurement value regardless of the device performance of the light receiving sensor 83, may clearly distinguish the measurement values of the whole blood and blood plasma because the difference between the measurement values of the whole blood and blood plasma is significant, and may have no need to set the respective correction values thereof in advance based on whether the whole blood or the blood plasma is sucked or not sucked.

The method of automatically distinguishing suction or non-suction of whole blood or blood plasma by using a reflective photosensor according to an exemplary embodiment of the present invention, which is improved for this purpose, in the automatic immunoassay device 1 including the round cartridge 100 which may simultaneously perform the centrifugation and automatic analysis of the blood sample and the tip which is movable up, down, left and right based on the round cartridge, includes: installing the reflective photosensor 80 below the round cartridge 100 and mounting the tip 61 above the round cartridge 100 (S10), and continuously measuring the blood non-suction data in a range including a position where the blood is sucked by using the reflective photosensor, collecting the measured data and storing the collected data while the tip 61 is raised (S20).

The method includes continuously measuring the blood suction data by using the reflective photosensor while the tip is lowered and absorbs the blood present in the range including the position where the blood is sucked from the round cartridge, collecting the measured data and storing the collected data (S30).

The method includes determining whether the type of the blood is the whole blood or the blood plasma, or whether the whole blood or the blood plasma is not sucked by comparing the blood non-suction data with the blood suction data (S40).

Here, the light source 81 of the reflective photosensor 80 is the long and thin light emitting diode (LED) disposed below the cartridge 100 in the axial direction of the cartridge 100, and the light receiving element 83 is the charge coupled device (CCD) linear image sensor disposed opposite to the light source 81, and installed in one row below the cartridge 100 in the axial direction of the cartridge 100.

In order to determine whether the type of the blood is the whole blood or the blood plasma, or whether the whole blood or the blood plasma is not sucked by comparing the blood non-suction data with the blood suction data, several measurements may be continuously performed to measure whether the whole blood or the blood plasma is not sucked into the same tip 61 used for one measurement, whether the whole blood or the blood plasma is not sucked into at least two or more different tips 61 used for different measurements, and whether the whole blood is sucked into the tip 61 and whether the blood plasma is sucked into the tip 61.

In order to determine whether the type of the blood is the whole blood or the blood plasma, or whether the whole blood or the blood plasma is not sucked by comparing the blood non-suction data with the blood suction data, the method of automatically distinguishing suction or non-suction of whole blood or blood plasma by using a reflective photosensor according to an exemplary embodiment of the present invention may use a slope of data obtained from the continuous measurements of the blood in a specific section.

Therefore, it is possible to reduce the deviation by using the slope of the data obtained from the continuous measurements of the blood rather than one fixed measurement value.

As shown in FIG. 6, the slope may be a relative ratio of the measurement value of the whole blood or blood plasma obtained by using the reflective photosensor 80 to the height of the tip 61.

The specific section may be a section including first and second inflection points of the values obtained from the continuous measurements of whether the whole blood (wb) and the (blood) plasma by using the reflective photosensor 80, i.e., the point at which a difference between the values obtained from the continuous measurements of the whole blood (wb) and the (blood) plasma by using the reflective photosensor 80 begins to occur and the point at which the slope of the values obtained from the continuous measurements of the whole blood (wb) and the (blood) plasma by using the reflective photosensor 80 has a maximum value.

In the specific section, the tip 61 may be disposed at a height of 10 steps or more and 46 steps or less when the total height is 200 steps, and 500 to 700 may be the maximum value of the difference between the measurement values of the whole blood and the blood plasma obtained by using the reflective photosensor.

Hereinafter, referring to FIGS. 7 through 12 and Tables 1-2 and 2-2, the description describes that the method of automatically distinguishing suction or non-suction of whole blood or blood plasma by using a reflective photosensor according to an exemplary embodiment of the present invention may provide the constant measurement value regardless of the device performance of the light receiving sensor 83, may clearly distinguish the measurement values of the whole blood and blood plasma because the difference between the measurement values of the whole blood and blood plasma is significant, and may have no need to set the respective correction values thereof in advance based on whether the whole blood or the blood plasma is sucked or not sucked.

FIGS. 7 to 10 are graphs showing whether the type of blood is the whole blood or the blood plasma and a result value of the whole blood or the blood plasma when the whole blood or the blood plasma is not sucked by comparing the blood non-suction data with the blood suction data, obtained from the same tip used for one measurement, in the automatic immunoassay device including the tip which may be moved up, down, left and right based on the round cartridge according to an exemplary embodiment of the present invention; and FIGS. 11 and 12 are graphs showing whether the type of the blood is the whole blood or the blood plasma and the result value of the whole blood or the blood plasma when the whole blood or the blood plasma is not sucked by comparing the blood non-suction data with the blood suction data, obtained from two different tips used for one measurement, in the automatic immunoassay device including the tip which may be moved up, down, left and right based on the round cartridge according to an exemplary embodiment of the present invention.

As shown in FIGS. 7 to 10, according to the method of automatically distinguishing suction or non-suction of whole blood or blood plasma by using a reflective photosensor according to an exemplary embodiment of the present invention, the values continuously measured by the four light receiving sensors 83, i.e. sensors M1, M2, M3 and M4, and the graphs of the values all show the same pattern.

That is, the method may provide the constant measurement value regardless of the device performance of the light receiving sensor 83, and may clearly distinguish the measurement values of the whole blood and blood plasma because the difference between the measurement values of the whole blood and blood plasma is significant.

**[Table 2-1]**

| | M1 | | | | M2 | | | | M3 | | | | M4 | | | |
|---|---|---|---|---|---|---|---|---|---|---|---|---|---|---|---|---|
| | tip | plsam | wb | tip2 | tip | plsam | wb | tip2 | tip | plsam | wb | tip2 | tip | plsam | wb | tip2 |
| 0 | 2164 | 2122 | 2123 | 2155 | 2074 | 2043 | 2045 | 2077 | 2204 | 2151 | 2120 | 2207 | 2270 | 2215 | 2206 | 2274 |
| 1 | 2163 | 2118 | 2125 | 2161 | 2071 | 2042 | 2046 | 2076 | 2204 | 2153 | 2120 | 2205 | 2270 | 2208 | 2206 | 2276 |
| 2 | 2165 | 2122 | 2123 | 2162 | 2069 | 2042 | 2045 | 2075 | 2205 | 2153 | 2122 | 2206 | 2269 | 2210 | 2206 | 2274 |
| 3 | 2164 | 2121 | 2123 | 2161 | 2070 | 2043 | 2046 | 2076 | 2204 | 2150 | 2122 | 2207 | 2270 | 2209 | 2207 | 2274 |
| 4 | 2168 | 2123 | 2122 | 2163 | 2070 | 2040 | 2047 | 2075 | 2204 | 2149 | 2123 | 2206 | 2268 | 2207 | 2204 | 2273 |
| 5 | 2166 | 2122 | 2122 | 2164 | 2067 | 2043 | 2046 | 2074 | 2201 | 2151 | 2123 | 2206 | 2270 | 2207 | 2202 | 2274 |
| 6 | 2166 | 2121 | 2120 | 2165 | 2067 | 2043 | 2044 | 2077 | 2203 | 2149 | 2127 | 2207 | 2269 | 2203 | 2200 | 2277 |
| 7 | 2166 | 2122 | 2119 | 2166 | 2069 | 2042 | 2045 | 2077 | 2201 | 2147 | 2128 | 2203 | 2270 | 2202 | 2195 | 2274 |
| 8 | 2168 | 2118 | 2117 | 2166 | 2068 | 2044 | 2046 | 2075 | 2203 | 2146 | 2130 | 2207 | 2271 | 2199 | 2191 | 2274 |
| 9 | 2166 | 2118 | 2108 | 2164 | 2069 | 2040 | 2044 | 2074 | 2203 | 2144 | 2128 | 2207 | 2271 | 2195 | 2181 | 2274 |
| 10 | 2168 | 2115 | 2103 | 2161 | 2071 | 2041 | 2041 | 2075 | 2202 | 2137 | 2127 | 2204 | 2273 | 2186 | 2177 | 2276 |
| 11 | 2168 | 2108 | 2091 | 2159 | 2070 | 2038 | 2036 | 2073 | 2201 | 2136 | 2124 | 2207 | 2271 | 2182 | 2159 | 2275 |
| 12 | 2167 | 2102 | 2071 | 2157 | 2071 | 2031 | 2030 | 2074 | 2201 | 2131 | 2116 | 2209 | 2271 | 2170 | 2139 | 2277 |
| 13 | 2169 | 2100 | 2070 | 2159 | 2069 | 2024 | 2017 | 2075 | 2200 | 2125 | 2107 | 2213 | 2271 | 2156 | 2128 | 2276 |
| 14 | 2169 | 2090 | 2039 | 2160 | 2070 | 2018 | 2011 | 2075 | 2199 | 2123 | 2100 | 2217 | 2273 | 2151 | 2108 | 2276 |
| 15 | 2171 | 2086 | 2021 | 2162 | 2069 | 2009 | 2000 | 2075 | 2199 | 2118 | 2084 | 2220 | 2272 | 2144 | 2090 | 2277 |
| 16 | 2169 | 2082 | 2012 | 2165 | 2068 | 2003 | 1986 | 2074 | 2198 | 2115 | 2062 | 2220 | 2271 | 2141 | 2081 | 2273 |
| 17 | 2169 | 2083 | 1997 | 2162 | 2066 | 2002 | 1978 | 2072 | 2197 | 2114 | 2056 | 2220 | 2267 | 2145 | 2065 | 2274 |
| 18 | 2167 | 2087 | 1985 | 2162 | 2068 | 2003 | 1968 | 2071 | 2197 | 2115 | 2031 | 2214 | 2265 | 2155 | 2052 | 2272 |
| 19 | 2166 | 2103 | 1978 | 2163 | 2065 | 2010 | 1958 | 2072 | 2196 | 2122 | 2013 | 2212 | 2264 | 2175 | 2046 | 2270 |
| 20 | 2166 | 2132 | 1970 | 2162 | 2065 | 2016 | 1952 | 2072 | 2194 | 2128 | 2007 | 2211 | 2266 | 2184 | 2036 | 2268 |
| 21 | 2166 | 2151 | 1967 | 2164 | 2066 | 2031 | 1945 | 2071 | 2195 | 2146 | 1994 | 2207 | 2259 | 2208 | 2027 | 2267 |
| 22 | 2165 | 2194 | 1965 | 2165 | 2064 | 2053 | 1938 | 2069 | 2193 | 2175 | 1987 | 2205 | 2261 | 2233 | 2021 | 2265 |
| 23 | 2167 | 2239 | 1959 | 2164 | 2065 | 2066 | 1936 | 2068 | 2192 | 2190 | 1990 | 2203 | 2259 | 2246 | 2016 | 2265 |
| 24 | 2165 | 2267 | 1956 | 2168 | 2063 | 2095 | 1933 | 2068 | 2194 | 2225 | 1975 | 2201 | 2260 | 2272 | 2009 | 2265 |
| 25 | 2168 | 2314 | 1953 | 2167 | 2065 | 2125 | 1930 | 2068 | 2192 | 2263 | 1969 | 2200 | 2262 | 2299 | 2007 | 2267 |
| 26 | 2168 | 2358 | 1950 | 2169 | 2061 | 2141 | 1928 | 2067 | 2191 | 2280 | 1965 | 2200 | 2261 | 2309 | 2002 | 2266 |
| 27 | 2168 | 2379 | 1945 | 2168 | 2061 | 2173 | 1924 | 2065 | 2193 | 2322 | 1957 | 2200 | 2261 | 2330 | 1998 | 2264 |
| 28 | 2167 | 2418 | 1944 | 2170 | 2060 | 2206 | 1922 | 2067 | 2191 | 2356 | 1955 | 2196 | 2258 | 2347 | 1994 | 2264 |
| 29 | 2166 | 2455 | 1940 | 2167 | 2058 | 2222 | 1921 | 2065 | 2190 | 2373 | 1951 | 2195 | 2256 | 2354 | 1991 | 2261 |
| 30 | 2165 | 2472 | 1939 | 2169 | 2057 | 2251 | 1919 | 2062 | 2189 | 2403 | 1949 | 2194 | 2251 | 2371 | 1986 | 2256 |
| 31 | 2164 | 2503 | 1939 | 2166 | 2058 | 2278 | 1918 | 2064 | 2188 | 2427 | 1944 | 2191 | 2251 | 2374 | 1984 | 2260 |
| 32 | 2162 | 2531 | 1939 | 2165 | 2060 | 2287 | 1916 | 2062 | 2186 | 2439 | 1946 | 2190 | 2251 | 2377 | 1987 | 2255 |
| 33 | 2162 | 2544 | 1940 | 2166 | 2057 | 2307 | 1917 | 2063 | 2189 | 2458 | 1945 | 2190 | 2245 | 2382 | 1985 | 2257 |
| 34 | 2164 | 2562 | 1940 | 2168 | 2058 | 2321 | 1915 | 2064 | 2191 | 2473 | 1946 | 2193 | 2251 | 2384 | 1987 | 2257 |
| 35 | 2161 | 2580 | 1945 | 2164 | 2059 | 2328 | 1914 | 2064 | 2191 | 2478 | 1948 | 2197 | 2253 | 2389 | 1990 | 2260 |
| 36 | 2162 | 2582 | 1946 | 2166 | 2061 | 2334 | 1917 | 2063 | 2192 | 2487 | 1949 | 2193 | 2253 | 2391 | 1993 | 2258 |
| 37 | 2163 | 2592 | 1946 | 2164 | 2060 | 2339 | 1919 | 2066 | 2190 | 2490 | 1953 | 2197 | 2254 | 2390 | 1994 | 2257 |
| 38 | 2165 | 2602 | 1950 | 2168 | 2060 | 2341 | 1919 | 2063 | 2193 | 2493 | 1955 | 2197 | 2253 | 2390 | 1999 | 2259 |
| 39 | 2165 | 2602 | 1955 | 2169 | 2058 | 2340 | 1918 | 2064 | 2194 | 2493 | 1951 | 2196 | 2253 | 2389 | 2002 | 2257 |
| 40 | 2164 | 2606 | 1954 | 2170 | 2059 | 2340 | 1920 | 2064 | 2194 | 2495 | 1956 | 2195 | 2251 | 2386 | 2000 | 2258 |
| 41 | 2164 | 2607 | 1957 | 2168 | 2058 | 2336 | 1921 | 2062 | 2196 | 2493 | 1958 | 2198 | 2250 | 2387 | 2005 | 2257 |
| 42 | 2165 | 2604 | 1960 | 2169 | 2059 | 2333 | 1922 | 2063 | 2196 | 2491 | 1960 | 2196 | 2248 | 2381 | 2006 | 2255 |
| 43 | 2164 | 2603 | 1957 | 2168 | 2059 | 2327 | 1924 | 2061 | 2197 | 2487 | 1963 | 2197 | 2249 | 2379 | 2008 | 2254 |
| 44 | 2164 | 2601 | 1963 | 2175 | 2060 | 2326 | 1924 | 2062 | 2193 | 2483 | 1964 | 2197 | 2249 | 2378 | 2011 | 2255 |
| 45 | 2167 | 2600 | 1963 | 2169 | 2061 | 2319 | 1924 | 2063 | 2196 | 2478 | 1965 | 2199 | 2249 | 2374 | 2013 | 2254 |
| 46 | 2166 | 2595 | 1965 | 2169 | 2061 | 2313 | 1926 | 2063 | 2198 | 2475 | 1967 | 2196 | 2250 | 2372 | 2015 | 2255 |
| 47 | 2169 | 2592 | 1968 | 2168 | 2060 | 2309 | 1923 | 2062 | 2196 | 2470 | 1968 | 2196 | 2251 | 2373 | 2018 | 2256 |
| 48 | 2169 | 2588 | 1968 | 2167 | 2060 | 2304 | 1927 | 2063 | 2195 | 2463 | 1971 | 2196 | 2253 | 2371 | 2022 | 2256 |
| 49 | 2167 | 2588 | 1967 | 2165 | 2061 | 2302 | 1926 | 2060 | 2193 | 2464 | 1972 | 2192 | 2256 | 2370 | 2024 | 2260 |

**[Table 2-2]**

| | | | | | | | | | | | | | | | | |
|---|---|---|---|---|---|---|---|---|---|---|---|---|---|---|---|---|
| 50 | 2167 | 2577 | 1968 | 2164 | 2060 | 2296 | 1928 | 2060 | 2193 | 2459 | 1974 | 2189 | 2256 | 2369 | 2027 | 2262 |
| 51 | 2167 | 2572 | 1968 | 2163 | 2060 | 2290 | 1929 | 2057 | 2194 | 2455 | 1975 | 2184 | 2254 | 2368 | 2028 | 2261 |
| 52 | 2167 | 2562 | 1969 | 2163 | 2062 | 2286 | 1931 | 2058 | 2191 | 2449 | 1976 | 2182 | 2258 | 2366 | 2026 | 2258 |
| 53 | 2166 | 2560 | 1969 | 2162 | 2062 | 2283 | 1930 | 2060 | 2190 | 2446 | 1978 | 2180 | 2259 | 2367 | 2030 | 2263 |
| 54 | 2166 | 2554 | 1969 | 2164 | 2062 | 2278 | 1932 | 2056 | 2189 | 2437 | 1978 | 2177 | 2260 | 2362 | 2031 | 2262 |
| 55 | 2168 | 2545 | 1971 | 2161 | 2061 | 2274 | 1933 | 2058 | 2189 | 2433 | 1981 | 2176 | 2259 | 2357 | 2034 | 2262 |
| 56 | 2167 | 2541 | 1971 | 2164 | 2061 | 2271 | 1933 | 2058 | 2190 | 2430 | 1980 | 2177 | 2258 | 2360 | 2036 | 2261 |
| 57 | 2170 | 2535 | 1971 | 2162 | 2060 | 2266 | 1933 | 2053 | 2189 | 2425 | 1984 | 2175 | 2260 | 2357 | 2035 | 2261 |
| 58 | 2168 | 2527 | 1971 | 2161 | 2062 | 2262 | 1937 | 2054 | 2190 | 2420 | 1986 | 2175 | 2257 | 2352 | 2036 | 2262 |
| 59 | 2172 | 2519 | 1973 | 2160 | 2063 | 2261 | 1936 | 2052 | 2187 | 2418 | 1988 | 2174 | 2260 | 2353 | 2038 | 2260 |
| 60 | 2170 | 2515 | 1975 | 2162 | 2062 | 2256 | 1937 | 2053 | 2187 | 2412 | 1992 | 2172 | 2257 | 2350 | 2041 | 2261 |
| 61 | 2170 | 2512 | 1975 | 2164 | 2062 | 2251 | 1938 | 2052 | 2186 | 2407 | 1992 | 2168 | 2258 | 2350 | 2041 | 2260 |
| 62 | 2171 | 2501 | 1974 | 2162 | 2065 | 2250 | 1938 | 2052 | 2184 | 2405 | 1992 | 2166 | 2258 | 2350 | 2045 | 2261 |
| 63 | 2173 | 2497 | 1976 | 2165 | 2063 | 2246 | 1940 | 2054 | 2184 | 2400 | 1995 | 2166 | 2261 | 2352 | 2049 | 2264 |
| 64 | 2173 | 2491 | 1977 | 2164 | 2063 | 2243 | 1942 | 2052 | 2184 | 2394 | 1995 | 2163 | 2262 | 2349 | 2050 | 2266 |
| 65 | 2173 | 2486 | 1980 | 2165 | 2065 | 2240 | 1940 | 2052 | 2183 | 2391 | 1996 | 2163 | 2266 | 2350 | 2055 | 2267 |
| 66 | 2175 | 2482 | 1979 | 2163 | 2066 | 2231 | 1944 | 2054 | 2183 | 2386 | 1996 | 2162 | 2267 | 2351 | 2058 | 2270 |
| 67 | 2174 | 2475 | 1979 | 2161 | 2066 | 2231 | 1942 | 2054 | 2182 | 2379 | 1996 | 2159 | 2269 | 2349 | 2060 | 2272 |
| 68 | 2175 | 2470 | 1979 | 2159 | 2069 | 2231 | 1941 | 2052 | 2181 | 2377 | 1997 | 2159 | 2269 | 2351 | 2063 | 2273 |
| 69 | 2176 | 2467 | 1982 | 2161 | 2067 | 2228 | 1946 | 2053 | 2180 | 2372 | 1995 | 2153 | 2271 | 2349 | 2067 | 2276 |
| 70 | 2177 | 2463 | 1985 | 2160 | 2070 | 2226 | 1948 | 2055 | 2178 | 2364 | 1993 | 2151 | 2270 | 2347 | 2068 | 2276 |
| 71 | 2176 | 2456 | 1983 | 2163 | 2068 | 2224 | 1949 | 2054 | 2177 | 2363 | 1994 | 2148 | 2275 | 2347 | 2070 | 2279 |
| 72 | 2177 | 2452 | 1984 | 2158 | 2070 | 2222 | 1951 | 2055 | 2177 | 2358 | 1994 | 2147 | 2276 | 2344 | 2072 | 2281 |
| 73 | 2176 | 2446 | 1983 | 2156 | 2073 | 2219 | 1951 | 2054 | 2177 | 2352 | 1994 | 2144 | 2275 | 2343 | 2073 | 2282 |
| 74 | 2175 | 2440 | 1988 | 2154 | 2074 | 2216 | 1952 | 2057 | 2177 | 2351 | 1995 | 2144 | 2279 | 2342 | 2076 | 2278 |
| 75 | 2177 | 2437 | 1987 | 2156 | 2074 | 2214 | 1954 | 2057 | 2177 | 2346 | 1995 | 2143 | 2282 | 2344 | 2080 | 2286 |
| 76 | 2177 | 2430 | 1990 | 2154 | 2075 | 2210 | 1956 | 2060 | 2177 | 2343 | 1996 | 2143 | 2284 | 2343 | 2081 | 2289 |
| 77 | 2178 | 2424 | 1989 | 2155 | 2078 | 2210 | 1954 | 2060 | 2180 | 2343 | 1995 | 2143 | 2286 | 2343 | 2086 | 2291 |
| 78 | 2179 | 2419 | 1991 | 2154 | 2080 | 2207 | 1959 | 2061 | 2176 | 2338 | 1998 | 2144 | 2288 | 2344 | 2089 | 2293 |
| 79 | 2176 | 2411 | 1991 | 2163 | 2079 | 2206 | 1962 | 2063 | 2176 | 2335 | 1998 | 2147 | 2290 | 2345 | 2099 | 2295 |
| 80 | 2177 | 2407 | 1989 | 2155 | 2082 | 2206 | 1961 | 2064 | 2176 | 2334 | 1998 | 2148 | 2290 | 2344 | 2094 | 2294 |
| 81 | 2177 | 2404 | 1991 | 2156 | 2081 | 2203 | 1963 | 2065 | 2176 | 2332 | 1999 | 2146 | 2291 | 2345 | 2094 | 2295 |
| 82 | 2181 | 2399 | 1992 | 2153 | 2080 | 2200 | 1965 | 2066 | 2173 | 2327 | 1999 | 2145 | 2293 | 2344 | 2097 | 2294 |
| 83 | 2175 | 2394 | 1990 | 2153 | 2084 | 2195 | 1965 | 2068 | 2172 | 2325 | 2000 | 2145 | 2295 | 2345 | 2100 | 2292 |
| 84 | 2175 | 2396 | 1993 | 2153 | 2085 | 2199 | 1965 | 2069 | 2172 | 2327 | 1999 | 2144 | 2295 | 2347 | 2101 | 2294 |
| 85 | 2177 | 2387 | 1993 | 2153 | 2087 | 2195 | 1969 | 2069 | 2172 | 2319 | 2001 | 2143 | 2297 | 2347 | 2103 | 2293 |
| 86 | 2177 | 2388 | 1996 | 2155 | 2086 | 2197 | 1969 | 2071 | 2172 | 2318 | 1999 | 2148 | 2300 | 2348 | 2107 | 2293 |
| 87 | 2182 | 2383 | 1997 | 2154 | 2087 | 2193 | 1971 | 2073 | 2171 | 2315 | 2002 | 2146 | 2302 | 2350 | 2111 | 2295 |
| 88 | 2179 | 2381 | 1996 | 2155 | 2088 | 2194 | 1971 | 2074 | 2169 | 2317 | 2002 | 2147 | 2306 | 2352 | 2114 | 2295 |
| 89 | 2183 | 2378 | 2000 | 2157 | 2091 | 2192 | 1975 | 2075 | 2173 | 2316 | 2003 | 2148 | 2308 | 2352 | 2117 | 2294 |
| 90 | 2181 | 2375 | 2002 | 2161 | 2092 | 2191 | 1977 | 2076 | 2173 | 2314 | 2009 | 2147 | 2310 | 2352 | *2122* | 2295 |
| 91 | 2181 | 2373 | 2001 | 2156 | 2093 | 2192 | 1979 | 2078 | 2173 | 2310 | 2004 | 2147 | 2312 | 2354 | 2124 | 2298 |
| 92 | 2184 | 2373 | 2005 | 2158 | 2096 | 2192 | 1979 | 2081 | 2167 | 2309 | 1999 | 2148 | 2313 | 2354 | 2126 | 2297 |
| 93 | 2184 | 2372 | 2007 | 2161 | 2095 | 2188 | 1981 | 2081 | 2170 | 2307 | 2005 | 2144 | 2316 | 2355 | 2134 | 2299 |
| 94 | 2187 | 2371 | 2006 | 2158 | 2100 | 2188 | 1985 | 2082 | 2168 | 2304 | 2006 | 2146 | 2317 | 2355 | 2133 | 2302 |
| 95 | 2187 | 2365 | 2009 | 2160 | 2099 | 2187 | 1984 | 2079 | 2168 | 2300 | 2006 | 2145 | 2319 | 2358 | 2139 | 2302 |
| 96 | 2187 | 2364 | 2008 | 2158 | 2101 | 2186 | 1987 | 2081 | 2167 | 2299 | 2006 | 2142 | 2321 | 2359 | 2142 | 2303 |
| 97 | 2188 | 2362 | 2009 | 2160 | 2103 | 2186 | 1989 | 2086 | 2166 | 2294 | 2007 | 2142 | 2323 | 2357 | 2145 | 2307 |
| 98 | 2190 | 2360 | 2012 | 2162 | 2105 | 2186 | 1991 | 2084 | 2166 | 2296 | 2007 | 2144 | 2325 | 2361 | 2149 | 2307 |
| 99 | 2192 | 2359 | 2013 | 2162 | 2105 | 2185 | 1989 | 2089 | 2165 | 2293 | 2007 | 2143 | 2327 | 2362 | 2151 | 2310 |

In addition, as shown in FIGS. 11 and 12, and Tables 3-1 and 3-1, it may be seen that a relative value may be used to analyze and determine the pattern because the different tips 61 have the measurement values slightly different from each other, but show the same pattern when analyzing whether the sample is the whole blood or the blood plasma or analyzing the measurement value of the whole blood or blood plasma when the whole blood or the blood plasma is not sucked by comparing the blood non-suction data with the blood suction data obtained from the different two tips used for one measurement by using the same reflective photosensor 80,in the automatic immunoassay device including the tip which may be moved up, down, left and right based on the round cartridge according to an exemplary embodiment of the present invention.

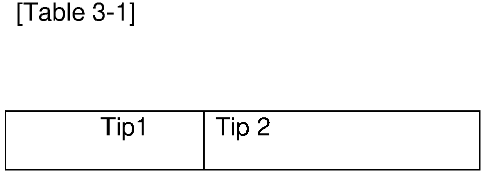

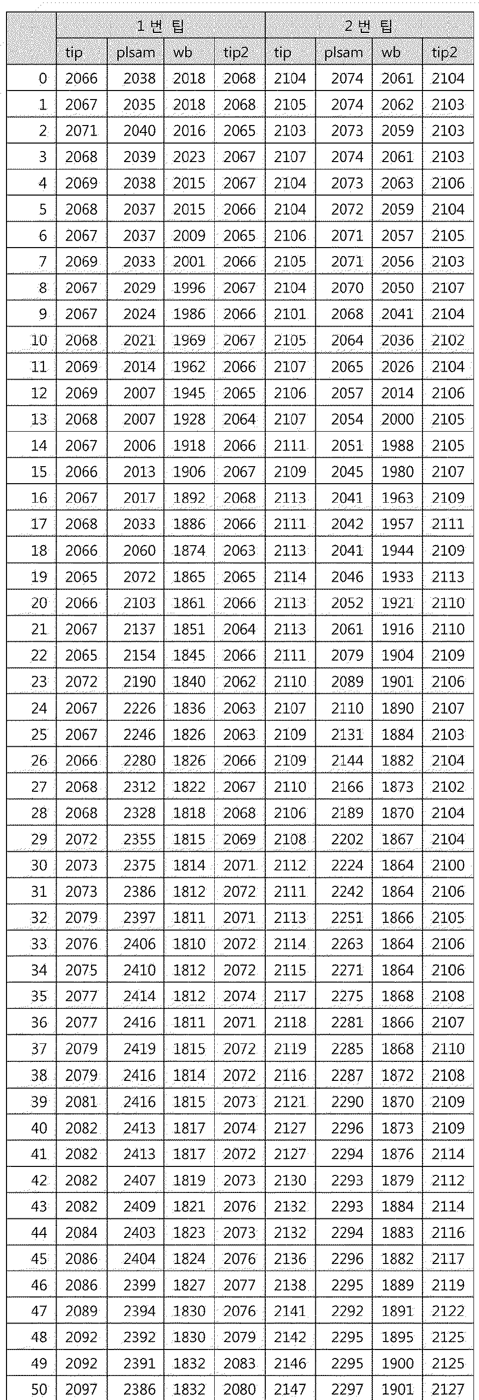

**[Table 3-2]**

| | | | | | | | | |
|---|---|---|---|---|---|---|---|---|
| 51 | 2095 | 2386 | 1835 | 2079 | 2150 | 2293 | 1904 | 2130 |
| 52 | 2097 | 2383 | 1836 | 2082 | 2153 | 2296 | 1908 | 2131 |
| 53 | 2096 | 2382 | 1839 | 2080 | 2158 | 2296 | 1910 | 2137 |
| 54 | 2099 | 2377 | 1840 | 2080 | 2159 | 2296 | 1914 | 2138 |
| 55 | 2097 | 2378 | 1836 | 2082 | 2166 | 2294 | 1918 | 2140 |
| 56 | 2098 | 2372 | 1843 | 2082 | 2167 | 2296 | 1917 | 2144 |
| 57 | 2098 | 2368 | 1845 | 2084 | 2173 | 2295 | 1925 | 2148 |
| 58 | 2101 | 2365 | 1849 | 2085 | 2175 | 2294 | 1932 | 2151 |
| 59 | 2106 | 2363 | 1851 | 2087 | 2182 | 2296 | 1936 | 2156 |
| 60 | 2108 | 2359 | 1856 | 2088 | 2183 | 2295 | 1939 | 2158 |
| 61 | 2112 | 2356 | 1859 | 2091 | 2188 | 2294 | 1946 | 2162 |
| 62 | 2112 | 2352 | 1859 | 2093 | 2194 | 2294 | 1948 | 2168 |
| 63 | 2118 | 2350 | 1863 | 2096 | 2196 | 2293 | 1956 | 2172 |
| 64 | 2119 | 2346 | 1868 | 2097 | 2201 | 2294 | 1960 | 2172 |
| 65 | 2125 | 2340 | 1871 | 2099 | 2210 | 2293 | 1964 | 2179 |
| 66 | 2127 | 2339 | 1874 | 2101 | 2208 | 2294 | 1970 | 2186 |
| 67 | 2129 | 2346 | 1877 | 2105 | 2215 | 2293 | 1974 | 2188 |
| 68 | 2134 | 2335 | 1879 | 2107 | 2218 | 2294 | 1977 | 2188 |
| 69 | 2139 | 2334 | 1884 | 2109 | 2222 | 2293 | 1983 | 2193 |
| 70 | 2137 | 2334 | 1886 | 2112 | 2224 | 2294 | 1989 | 2194 |
| 71 | 2143 | 2330 | 1889 | 2110 | 2230 | 2295 | 1991 | 2199 |
| 72 | 2146 | 2327 | 1894 | 2117 | 2233 | 2293 | 1996 | 2202 |
| 73 | 2146 | 2327 | 1895 | 2119 | 2236 | 2292 | 2001 | 2205 |
| 74 | 2150 | 2328 | 1895 | 2119 | 2242 | 2293 | 2005 | 2207 |
| 75 | 2152 | 2322 | 1900 | 2121 | 2241 | 2295 | 2007 | 2213 |
| 76 | 2153 | 2323 | 1905 | 2122 | 2244 | 2294 | 2015 | 2214 |
| 77 | 2158 | 2318 | 1906 | 2125 | 2251 | 2297 | 2016 | 2219 |
| 78 | 2159 | 2319 | 1912 | 2125 | 2253 | 2296 | 2023 | 2221 |
| 79 | 2160 | 2317 | 1912 | 2130 | 2256 | 2296 | 2030 | 2226 |
| 80 | 2165 | 2316 | 1915 | 2130 | 2262 | 2298 | 2029 | 2230 |
| 81 | 2166 | 2314 | 1917 | 2132 | 2268 | 2297 | 2036 | 2234 |
| 82 | 2169 | 2316 | 1923 | 2136 | 2273 | 2301 | 2043 | 2236 |
| 83 | 2174 | 2312 | 1926 | 2137 | 2277 | 2302 | 2048 | 2241 |
| 84 | 2179 | 2313 | 1932 | 2142 | 2281 | 2302 | 2054 | 2247 |
| 85 | 2180 | 2309 | 1936 | 2145 | 2287 | 2306 | 2062 | 2247 |
| 86 | 2183 | 2307 | 1937 | 2146 | 2293 | 2310 | 2066 | 2255 |
| 87 | 2188 | 2306 | 1944 | 2152 | 2294 | 2308 | 2074 | 2260 |
| 88 | 2189 | 2307 | 1948 | 2154 | 2304 | 2312 | 2083 | 2266 |
| 89 | 2194 | 2303 | 1951 | 2155 | 2309 | 2313 | 2084 | 2271 |
| 90 | 2200 | 2304 | 1959 | 2164 | 2313 | 2316 | 2092 | 2274 |
| 91 | 2203 | 2305 | 1963 | 2169 | 2321 | 2319 | 2098 | 2280 |
| 92 | 2206 | 2309 | 1965 | 2170 | 2324 | 2323 | 2102 | 2286 |
| 93 | 2211 | 2308 | 1971 | 2177 | 2329 | 2320 | 2108 | 2295 |
| 94 | 2215 | 2305 | 1977 | 2177 | 2337 | 2324 | 2115 | 2296 |
| 95 | 2217 | 2301 | 1973 | 2179 | 2339 | 2328 | 2119 | 2305 |
| 96 | 2224 | 2307 | 1985 | 2182 | 2341 | 2330 | 2124 | 2310 |
| 97 | 2224 | 2307 | 1987 | 2185 | 2347 | 2332 | 2131 | 2311 |
| 98 | 2231 | 2304 | 1989 | 2186 | 2351 | 2334 | 2133 | 2316 |
| 99 | 2233 | 2306 | 1995 | 2190 | 2353 | 2336 | 2143 | 2320 |

### [Industrial Applicability]

The method of automatically distinguishing the suction or non-suction of the whole blood or the blood plasma by using the reflective photosensor according to an exemplary embodiment of the present invention may accurately check whether the whole blood or the blood plasma is sucked or whether the whole blood or the blood plasma is not sucked from the round cartridge into the pipette mounted on the tip of the automatic immunoassay device including the round cartridge which may simultaneously perform the centrifugation and automatic analysis of the blood sample and the tip which is movable up, down, left and right based on the round cartridge.

## Claims

1. A method of automatically distinguishing whether a type of the blood is whole blood or blood plasma, or whether the whole blood or the blood plasma is not sucked into a tip (61) by using a reflective photosensor (80) in an automatic immunoassay device (1), the automatic immunoassay device (1) including a rotary drive unit (50) and a round cartridge (100) having wells for simultaneously performing centrifugation and automatic analysis of a blood sample therein and a tip (61) being movable up, down, left and right relative to the round cartridge (100) and configured to suck whole blood or blood plasma from the wells of the round cartridge (100),
the reflective photosensor (80) including a light source (81) which is a long and thin light emitting diode, LED, disposed below the round cartridge (100) in an axial direction of the cartridge (100), and a light receiving element (83) which is a charge coupled device, CCD, linear image sensor opposite to the light source (81), and installed in one row below the cartridge (100) in the axial direction of the cartridge (100), the method comprising:
installing the reflective photosensor (80) below the round cartridge (100) so the light receiving element (83) receives light from the light source (81) and reflected on a surface of the wells;
mounting the tip (61) above the round cartridge (100), and continuously measuring blood non-suction data in a range including a position where the blood is sucked by using the reflective photosensor (80), collecting the measured data and storing the collected data while the tip (61) is raised;
continuously measuring blood suction data by using the reflective photosensor (80) while the tip (61) is lowered and sucks the blood present in the range including the position where the blood is sucked from the round cartridge (100); and
determining whether a type of the blood is whole blood or blood plasma, or whether the whole blood or the blood plasma is not sucked by comparing the blood non-suction data with the blood suction data, wherein the determining uses a slope, the slope being a ratio of the measurement value of the whole blood or blood plasma obtained by using the reflective photosensor (80) to a height of the tip (61).

2. The method of claim 1, wherein
the determining of whether the type of the blood is the whole blood or the blood plasma, or whether the whole blood or the blood plasma is not sucked by comparing the blood non-suction data with the blood suction data includes performing the measurements of whether the whole blood or the blood plasma is not sucked into the tip (61) or at least two or more different tips, whether the whole blood is sucked into the tip (61), and whether the blood plasma is sucked into the tip (61).

3. The method of claim 1, wherein
in the determining of whether the type of the blood is the whole blood or the blood plasma, or whether the whole blood or the blood plasma is not sucked by comparing the blood non-suction data with the blood suction data,
the determination is made by the slope of data obtained from the continuous measurements of the blood in a section including
a point at which a difference between values obtained from the continuous measurements of the whole blood (wb) and the blood plasma by using the reflective photosensor (80) begins to occur and
a point at which the slope of the values obtained from the continuous measurements of the whole blood (wb) and the blood plasma by using the reflective photosensor (80) has a maximum value.

4. The method of claim 3, wherein
the tip (61) in the section has a height of 10 steps or more and 46 steps or less when a total height is 200 steps.

5. The method of claim 3, wherein
500 to 700 is a maximum value of a difference between the measurement values of the whole blood and the blood plasma obtained by using the reflective photosensor (80).

## Patentansprüche

1. Verfahren zur automatischen Unterscheidung, ob es sich bei einer Blutart um Vollblut oder Blutplasma handelt oder ob das Vollblut oder das Blutplasma nicht in eine Spitze (61) gesaugt wurde, mittels eines reflektierenden Fotosensors (80) in einer automatischen Immunoassay-Vorrichtung (1), wobei die automatische Immunoassay-Vorrichtung (1) eine rotierende Antriebseinheit (50) und eine runde Kartusche (100) mit Vertiefungen zur gleichzeitigen Durchführung von Zentrifugation und automatischer Analyse einer darin enthaltenen Blutprobe sowie eine Spitze (61) beinhaltet, die relativ zur runden Kartusche (100) auf, ab, links und rechts beweglich ist und so konfiguriert ist, dass sie Vollblut oder Blutplasma aus den Vertiefungen der runden Kartusche (100) ansaugt.
wobei der reflektierende Fotosensor (80) eine Lichtquelle (81), die eine lange und dünne Leuchtdiode, LED, ist, die unterhalb der runden Kartusche (100) in Axialrichtung der Kartusche (100) angeordnet ist, und ein Lichtempfangselement (83) beinhaltet, das eine ladungsgekoppelte lineare Bildsensorvorrichtung, CCD, ist, die der Lichtquelle (81) gegenüberliegt und in einer Reihe unterhalb der Kartusche (100) in der axialen Richtung der Kartusche (100) installiert ist, wobei das Verfahren umfasst:
Installieren des reflektierenden Fotosensors (80) unterhalb der runden Kartusche (100) so, dass das Lichtempfangselement (83) Licht von der Lichtquelle (81) empfängt, das auf eine Oberfläche der Vertiefungen reflektiert wird;
Montieren der Spitze (61) über der runden Kartusche (100) und kontinuierliches Messen von Daten ohne Blutansaugung in einem Bereich, der eine Position beinhaltet, an der das Blut angesaugt wird, mittels des reflektierenden Fotosensors (80), Sammeln der gemessenen Daten und Speichern der gesammelten Daten, während die Spitze (61) angehoben ist;
kontinuierliches Messen der Daten mit Blutansaugung mittels des reflektierenden Fotosensors (80), während die Spitze (61) abgesenkt ist und das Blut ansaugt, das im Bereich einschließlich der Position, an der das Blut aus der runden Kartusche (100) angesaugt wird, vorhanden ist; und
Bestimmen, ob es sich bei einer Blutart um Vollblut oder Blutplasma handelt oder ob das Vollblut oder das Blutplasma nicht angesaugt wurde, durch Vergleichen der Daten ohne Blutansaugung mit den Daten mit Blutansaugung, wobei das Bestimmen mittels einer Steigung erfolgt, wobei die Steigung ein Verhältnis des mit dem reflektierenden Fotosensor (80) gewonnenen Messwerts für Vollblut oder Blutplasma zur Höhe der Spitze (61) ist.

2. Verfahren nach Anspruch 1, wobei
das Bestimmen, ob es sich bei der Blutart um Vollblut oder Blutplasma handelt oder ob das Vollblut oder das Blutplasma nicht angesaugt wurde, durch Vergleichen der Daten ohne Blutansaugung mit den Daten mit Blutansaugung Durchführen der Messungen beinhaltet, ob das Vollblut oder Blutplasma nicht in die Spitze (61) oder mindestens zwei oder mehr verschiedene Spitzen angesaugt wird, ob das Vollblut in die Spitze (61) angesaugt wird und ob das Blutplasma in die Spitze (61) angesaugt wird.

3. Verfahren nach Anspruch 1, wobei
beim Bestimmen, ob es sich bei der Blutart um Vollblut oder Blutplasma handelt oder ob das Vollblut oder das Blutplasma nicht angesaugt wurde, durch Vergleichen der Daten ohne Blutansaugung mit den Daten mit Blutansaugung,
das Bestimmen anhand der Steigung der Daten erfolgt, die aus den kontinuierlichen Messungen des Blutes in einem Abschnitt gewonnen werden, beinhaltend
einen Punkt, an dem ein Unterschied zwischen den Werten, die aus den kontinuierlichen Messungen des Vollbluts (wb) und des Blutplasmas mittels des reflektierenden Fotosensors (80) gewonnen werden, auftritt und
einen Punkt, an dem die Steigung der Werte, die aus den kontinuierlichen Messungen des Vollbluts (wb) und des Blutplasmas mittels des reflektierenden Fotosensors (80) gewonnen werden, einen Maximalwert aufweist.

4. Verfahren nach Anspruch 3, wobei
die Spitze (61) in dem Abschnitt eine Höhe von 10 Stufen oder mehr und 46 Stufen oder weniger hat, wenn die Gesamthöhe 200 Stufen beträgt.

5. Verfahren nach Anspruch 3, wobei
500 bis 700 ein Maximalwert der Differenz zwischen den Messwerten des Vollbluts und des Blutplasmas, die mittels des reflektierenden Fotosensors (80) gewonnen wurden.

## Revendications

1. Procédé permettant de distinguer automatiquement si un type du sang est du sang total ou du plasma sanguin, ou si le sang total ou le plasma sanguin n'est pas aspiré dans un embout (61), en utilisant un photocapteur réfléchissant (80) dans un dispositif d'immunoanalyse automatique (1), le dispositif d'immunoanalyse automatique (1) incluant une unité d'entraînement rotative (50) et une cartouche ronde (100) comportant des puits pour effectuer simultanément une centrifugation et une analyse automatique d'un échantillon de sang dans celui-ci, et un embout (61) qui peut se déplacer vers le haut, vers le bas, à gauche, à droite par rapport à la cartouche ronde (100) et configurée pour aspirer le sang total ou le plasma sanguin à partir des puits de la cartouche ronde (100),
le photocapteur réfléchissant (80) incluant une source lumineuse (81) qui est une diode électroluminescente, DEL, longue et mince, disposée sous la cartouche ronde (100) dans une direction axiale de la cartouche (100), et un élément de réception de lumière (83) qui est un capteur d'image linéaire de dispositif à couplage de charge, CCD, opposé à la source lumineuse (81), et installé sur une rangée sous la cartouche (100) dans la direction axiale de la cartouche (100), le procédé comprenant :
l'installation du photocapteur réfléchissant (80) sous la cartouche ronde (100) de sorte que l'élément de réception de lumière (83) reçoive la lumière en provenance de la source lumineuse (81) et réfléchie sur une surface des puits ;
le montage de l'embout (61) au-dessus de la cartouche ronde (100) et la mesure en continu de données de non-aspiration du sang dans une plage incluant une position dans laquelle le sang est aspiré en utilisant le photocapteur réfléchissant (80), la collecte des données mesurées et le stockage des données collectées pendant que l'embout (61) est relevé ;
la mesure en continu des données d'aspiration du sang en utilisant le photocapteur réfléchissant (80) pendant que l'extrémité (61) est abaissée et aspire le sang présent dans la plage, incluant la position dans laquelle le sang est aspiré depuis la cartouche ronde (100) ; et
la détermination pour savoir si un type de sang est du sang total ou du plasma sanguin, ou si le sang total ou le plasma sanguin n'est pas aspiré en comparant les données de non-aspiration du sang avec les données d'aspiration du sang, dans lequel la détermination utilise une pente, la pente étant un rapport de la valeur de mesure du sang total ou du plasma sanguin obtenue en utilisant le photocapteur réfléchissant (80) à une hauteur de l'embout (61).

2. Procédé selon la revendication 1, dans lequel
la détermination pour savoir si le type du sang est le sang total ou le plasma sanguin ou si le sang total ou le plasma sanguin n'est pas aspiré en comparant les données de non-aspiration du sang avec les données d'aspiration du sang inclut la réalisation des mesures pour savoir si le sang total ou le plasma sanguin n'est pas aspiré dans l'embout (61) ou dans au moins deux, ou plus, embouts différents, si le sang total est aspiré dans l'embout (61) et si le plasma sanguin est aspiré dans l'embout (61).

3. Procédé selon la revendication 1, dans lequel
lors de la détermination pour savoir si le type de sang est le sang total ou le plasma sanguin, ou si le sang total ou le plasma sanguin n'est pas aspiré, en comparant les données de non-aspiration du sang avec les données d'aspiration du sang,
la détermination est réalisée à partir de la pente des données obtenues à partir des mesures continues du sang dans une section incluant
un point auquel une différence entre des valeurs obtenues à partir des mesures continues du sang total (wb) et du plasma sanguin en utilisant le photocapteur réfléchissant (80) commence à se produire, et
un point auquel la pente des valeurs obtenues à partir des mesures continues du sang total (wb) et du plasma sanguin en utilisant le photocapteur réfléchissant (80) présente une valeur maximale.

4. Procédé selon la revendication 3, dans lequel
l'embout (61) dans la section présente une hauteur de 10 marches ou plus et de 46 marches ou moins lorsqu'une hauteur totale est 200 marches.

5. Procédé selon la revendication 3, dans lequel
500 à 700 est une valeur maximale de différence entre les valeurs de mesure du sang total et du plasma sanguin obtenues en utilisant le photocapteur réfléchissant (80).
